# EUROPÄISCHE PATENTSCHRIFT

(11) **EP 2 874 548 B1**
(45) Veröffentlichungstag und Bekanntmachung des Hinweises auf die Patenterteilung: **01.06.2016**
(21) Anmeldenummer: 13736817.1
(22) Anmeldetag: 01.07.2013
(51) Int. Cl.: A61B 17/29, A61B 90/00

(54) **ENDOSKOPISCHES INSTRUMENT**
ENDOSCOPIC INSTRUMENT
INSTRUMENT ENDOSCOPIQUE

(30) Priorität: 17.07.2012 DE 102012212510
(43) Veröffentlichungstag der Anmeldung: 27.05.2015
(73) Patentinhaber: Richard Wolf GmbH, 75438 Knittlingen (DE)
(72) Erfinder: PRESTEL, Stephan, 76287 Rheinstetten (DE)
(74) Vertreter: Patentanwälte Vollmann & Hemmer
(86) Internationale Anmeldenummer: PCT/EP2013/063865
(87) Internationale Veröffentlichungsnummer: WO 2014/012780

(56) Entgegenhaltungen:
- US-A- 5 562 702
- US-A1- 2003 135 204
- US-A1- 2012 158 013
- US-B1- 6 312 435
- US-B1- 6 394 998

## Beschreibung

Die Erfindung betrifft ein endoskopisches Instrument gemäß den im O-berbegriff des Anspruchs 1 angegebenen Merkmalen.

Endoskopische Instrumente dieser Art, welche einen Instrumentenkopf mit einem Werkzeug mit zwei zueinander schwenkbaren Maulteilen aufweisen, werden heutzutage vielfältig eingesetzt, beispielsweise als Scheren, Zangen oder dergleichen. Dabei erfolgt die Bewegung der Maulteile durch Steuerung vom proximalen Instrumentenende aus, entweder über eine Handhabe manuell oder robotisch, d. h. durch eine entsprechende Steuerungsvorrichtung.

Derartige Instrumente sind beispielsweise aus US 6,312,435 B1, US 6,371,952 B1, US 6,206,903 B1, US 2007/0208375 A1, US 2011/0106145 A1, US 2012/0158013 A1 oder WO 2010/005657 A2 bekannt.

Ungeachtet ob diese robotisch, d. h. mit elektromotorischer Ansteuerung oder aber manuell betätigt werden, ist ein wesentliches Kriterium für den Einsatz der Schaftdurchmesser. Je kleiner der Schaftdurchmesser ist, desto vielfältiger ist das Instrument einzusetzen, es kann durch enge natürliche oder durch den Operateur geschaffene Öffnungen zum Operationsfeld geführt werden.

Ein Nachteil dünner Schaftdurchmesser und damit einhergehend in der Regel auch dünner Instrumentenköpfe ist es, dass es konstruktiv schwierig ist, die erforderlichen Kräfte durch Betätigung am proximalen Ende im Werkzeug aufzubringen, dies um so mehr, wenn, was häufig erforderlich ist, der Instrumentenkopf schwenkbar gegenüber dem Schaft sein soll.

Vor diesem Stand der Technik (US 2012/0158013 A1) liegt der Erfindung die Aufgabe zugrunde, ein gattungsgemäßes endoskopisches Instrument so auszubilden, dass einerseits ein möglichst kleiner Schaftdurchmesser realisierbar ist, andererseits jedoch die auf das Werkzeug, insbesondere die Maulteile zu übertragenden Kräfte möglichst hoch sind. Des Weiteren soll das Instrument konstruktiv einfach im Aufbau und kostengünstig in der Herstellung sein.

Diese Aufgabe wird gemäß der Erfindung durch ein endoskopisches Instrument mit den in Anspruch 1 angegebenen Merkmalen gelöst. Vorteilhafte Ausgestaltungen der Erfindung ergeben sich aus den Unteransprüchen, der nachfolgenden Beschreibung und der Zeichnung.

Das erfindungsgemäße endoskopische Instrument weist einen Schaft auf, an dessen distalen Ende ein Instrumentenkopf angeordnet ist, welcher ein Werkzeug mit vorzugsweise zwei zueinander schwenkbaren Maulteilen aufweist. Innerhalb des Schaftes sind Zugmittel vom proximalen Instrumentenende aus steuerbar, wobei jedes Maulteil zwei Kurvenscheiben aufweist, an denen je ein Zugmittel angreift. Gemäß der Erfindung sind jedem Maulteil zwei Kurvenscheiben unterschiedlicher radialer Größe zugeordnet, wobei jeweils die radial größere Kurvenscheibe näher der Längsmittelachse des Werkzeugs angeordnet ist als die radial kleinere Kurvenscheibe.

Grundgedanke der erfindungsgemäßen Lösung ist, Kurvenscheiben unterschiedlicher radialer Größen zu verwenden und dabei die radial größeren Kurvenscheiben zentral nahe der Werkzeugachse und die radial kleineren weiter außen anzuordnen, um auf diese Weise den in der Regel sich auch im Werkzeugkopf fortsetzenden runden Schaftquerschnitt möglichst optimal auszunutzen. Dabei ist für die auf das jeweilige Maulteil aufzubringende Kraft der radiale Abstand der Kurvenscheibe zur Drehachse entscheidend, da dieser bei vorgegebener Kraft das Moment bestimmt, mit welchem das Maulteil bewegt werden kann. Dadurch ist beispielsweise bei einer Zange die maximale Schließkraft bestimmt oder bei einer Schere die Scherkraft. Durch die erfindungsgemäße Anordnung wird erreicht, dass in eine Schwenkrichtung eines Maulteils eine höhere Kraft als in andere Richtung aufgebracht werden kann, nämlich dort, wo die radial größere Kurvenscheibe Verwendung findet. In welche Richtung diese Kraft aufgebracht werden soll, ist im Wesentlichen vom Verwendungszweck abhängig. Soll beispielsweise mit den Maulteilen ein Hohlraum offengehalten oder gespreizt werden, so ist es zweckmäßig die radial größeren Kurvenscheiben zum Schwenken der Maulteile in Öffnungsstellung vorzusehen. Bei einer Zange oder Schere hingegeben, wird zweckmäßigerweise die höhere Kraft in Schließrichtung benötigt, weshalb es dann von Vorteil ist, die radial größeren Kurvenscheiben den Zugmitteln zuzuordnen, welche die Maulteile aufeinander zu bewegen.

Bei der vorbeschriebenen bevorzugten erfindungsgemäßen Lösung, bei welcher jedem Maulteil zwei Kurvenscheiben zugeordnet sind, kann neben dem Schließen und Öffnen der Maulteile im Weiteren eine Schwenkbewegung realisiert werden, welche einen zusätzlichen Freiheitsgrad gewährt, wenn beide Maulteile gleichzeitig in dieselbe Richtung geschwenkt werden.

Es versteht sich, dass das erfindungsgemäße Prinzip in einfachster Form mit einem schwenkbaren Maulteil und einem weiteren feststehenden Maulteil realisiert werden kann, dann sind für das schwenkbare Maulteil zwei Kurvenscheiben vorgesehen, nämlich eine radial größere Kurvenscheibe, welche nahe der Längsmittelachse oder im Bereich der Längsmittelachse des Werkzeugs angeordnet und eine radial kleinere Kurvenscheibe, welche daneben angeordnet ist, dort wo weniger Raum innerhalb des Schaftquerschnitts zur Verfügung steht.

Bei Werkzeugen wie Zangen, Scheren und dergleichen, welche ihre größte Kraft in Schließrichtung aufbringen müssen, ist es vorteilhaft, wenn die radial größeren Kurvenscheiben den Zugmitteln zum Schließen und die radial kleineren Kurvenscheiben den Zugmitteln zum Öffnen der Maulteile zugeordnet sind. Die Öffnungskräfte können in der Regel um ein Vielfaches geringer sein, als die erforderlichen Schließkräfte, weshalb hier kleine Hebel, d. h. kleine Durchmesser der Kurvenscheibe ausreichen, um die erforderlichen Kräfte aufzubringen. Diese Kurvenscheiben, an denen die Zugmittel zum Öffnen der Maulteile angeordnet sind, können einen vergleichsweise kleinen Durchmesser haben und damit weiter außen angeordnet werden, während die radial größeren Kurvenscheiben, also die deren Kurvenbahn einen größeren radialen Abstand von der Drehachse aufweist, zentrumsnah angeordnet sind, dort wo der größte Freiraum bei den üblichen runden oder ovalen Instrumentendurchmesser gegeben ist.

Grundsätzlich ist die Form der Kurvenscheiben frei wählbar. So kann beispielsweise durch eine elliptische Kurvenscheibe die Kraft beim Maulteil in bestimmten Stellungen gezielt erhöht werden. Dies kann beispielsweise sinnvoll sein, wenn nur ein Maulteil schwenkbeweglich ist, um die höchste Kraft unmittelbar vor oder in der Schließstellung zu erzielen. Bei der bevorzugten Ausführung, bei welcher beide Maulteile in beiden Richtungen schwenkbar sind und somit einen weiteren Freiheitsgrad des endoskopischen Instrumenten realisieren, ist es zweckmäßig und vorteilhaft, die Kurvenscheiben kreisförmig auszubilden und vorzugsweise mit ihrem Kreismittelpunkt auf der gemeinsamen Schwenkachse der Maulteile anzuordnen. Eine solche Anordnung gewährleistet bei vorgegebener Kraft am Zugmittel unabhängig von der Schwenkstellung stets das gleiche Moment am Maulteil, was grundsätzlich vorteilhaft ist.

Unter radialer Größe im Sinne der vorliegenden Erfindung ist der maximale radiale Abstand der Kurvenscheibe von ihrem Drehpunkt zu verstehen. D. h. die größere Kurvenscheibe ist stets die, die den größeren radialen Abstand von ihrem Drehpunkt aufweist, auch wenn diese möglicherweise schmaler ist oder in Teilbereichen einen geringeren radialen Abstand aufweist, als die andere Kurvenscheibe. Der maximale radiale Abstand bestimmt den erforderlichen Freiraum, der zur Anordnung der Kurvenscheibe erforderlich ist. Hier soll gemäß der Erfindung die größere Kurvenscheibe in der Längsmittelachse oder nahe der Längsmittelachse angeordnet sein und die kleinere weiter außen daneben.

Vorteilhaft sind gemäß einer Weiterbildung der Erfindung die Zugmittel nicht um die Kurvenscheiben umlaufend sondern endseitig dort festgelegt und weisen einen Umschlingungswinkel von vorteilhaft mehr als 90° auf. Der Umschlingungswinkel von mehr als 90° gewährleistet, dass das jeweilige Maulteil auch um einen Winkel von 90° oder mehr schwenkbar ist, was von Vorteil ist.

Konstruktiv besonders günstig ist es, wenn ein Maulteil einstückig mit den zwei zugehörigen Kurvenscheiben ausgebildet ist. Hierdurch ist einerseits die erforderliche Momentenübertragung gewährleistet, andererseits kann ein solches Bauteil kostengünstig in einem Guss- oder anderem formgebenden Verfahren hergestellt werden.

Dabei ist es besonders vorteilhaft, wenn die beiden Maulteile identisch ausgebildet sind, wie dies beispielhaft im Ausführungsbeispiel weiter unten beschrieben ist. Eine solche Ausbildung hat den Vorteil, dass das Werkzeug mit zwei identischen Bauteilen aufgebaut werden kann, was die Herstellungskosten und Lagerhaltung verringert.

Um dem Instrument einen weiteren Freiheitsgrad hinsichtlich der Bewegbarkeit des Instrumentenkopfes zu geben, ist gemäß einer vorteilhaften Weiterbildung der Erfindung ein Zwischenstück vorgesehen, in welchem die Maulteile gelagert sind und welches Teil des Instrumentenkopfes bildet und schwenkbar am distalen Schaftende angeordnet ist. Ein solches Zwischenstück kann entweder die Schwenkbarkeit der Maulteile weiter erhöhen, wenn beispielsweise die Schwenkachse des Zwischenstückes parallel zur Schwenkachse der Maulteile ist. Besonders vorteilhaft wird es jedoch in der Regel sein, wenn die Schwenkachse des Zwischenstücks mit Abstand und quer zur Schwenkachse der Maulteile angeordnet ist, wodurch eine Schwenkbewegung des Instrumentenkopfes quer zur Schwenkachse des Werkzeugs möglich ist, was die Freiheitsgrade bei der Bewegung des Werkzeugskopfes erhöht und somit die Vielseitigkeit des Instrumentes bei der Anwendung verbessert.

Vorteilhaft ist gemäß einer Weiterbildung der Erfindung vorgesehen, dass das Zwischenstück aus einem Grundkörper aufgebaut ist, von dem sich zwei zueinander beabstandete Schenkel distalwärts erstrecken, welche die Maulteile mit ihrer Schwenkachse aufnehmen und von dem sich in Achsrichtung als in Richtung der Längsachse um 90° versetzt dazu zwei zueinander beabstandete Schenkel proximalwärts erstrecken, welche die Schwenkachse des Zwischenstücks und die drauf angeordneten Bauteile aufnehmen. Dabei weist der Grundkörper typischerweise einer kreisrunde Außenkontur auf, welche der Schaftkontur vorteilhaft entspricht, wohingegen die Schenkel der Schaftkontur folgend jeweils an zwei etwa um 180° versetzten Stellen nahe dem Außenumfang des Grundkörper ansetzen.

Um das Zwischenstück gegenüber dem Instrumentenschaft in seiner Schwenkbewegung mit Zugmitteln steuern zu können, ist es vorteilhaft, wenn die proximalwärts gerichteten Schenkel des Zwischenstücks drehfest mit einer Welle verbunden sind, auf der wiederum drehfest eine Kurvenscheibe angeordnet ist, an welcher Zugmittel zur Steuerung der Schwenkstellung des Zwischenstücks angreifen, welche durch den Schaft zum proximalen Instrumentenende führen. Dabei können mit der Welle ein oder zwei Kurvenscheiben verbunden sein, je nachdem ob für jedes Zugmittel eine Kurvenscheibe vorgesehen werden soll oder, was besonders vorteilhaft ist, beide Zugmittel an einer Kurvenscheibe angreifen, die dann mittig innerhalb des Bereiches der Längsachse angeordnet werden kann, in welchem aufgrund der Querschnittskontur der größte Freiraum gegeben ist.

Um die Zugmittel zur Bewegung der Maulteile durch das Gelenk am Instrumentenkopf zu führen, ist es vorteilhaft zwischen dem proximalwärts gerichteten Schenkeln für jedes zu einem Maulteil führenden Zugmittel ein Umlenkrollenpaar drehbar gelagert anzuordnen, und zwar so, dass eine der Umlenkrollen auf der Welle, deren Achse die Schwenkachse des Gelenks bildet und die andere versetzt dazu zwischen Welle und Grundkörper angeordnet ist. Eine solche Umlenkrollenführung ist von der Kraftübertragung besonders günstig, da die durch das Zugmittel aufgebrachte Kraft mit nur wenigen Verlusten durch das Gelenk hindurch zu den Maulteilen übertragen werden kann. Eine solche Rollenführung ist stets reibungsärmer als z. B. eine Bowdenzuganordnung. Sie kann darüber hinaus auch über Schwenkbereiche Verwendung finden, die über 90° hinausgehen.

Eine besonders vorteilhafte Weiterbildung des erfindungsgemäßen Instrumentes ergibt sich, wenn jedes Umlenkrollenpaar so angeordnet wird, dass der Umschlingungswinkel des zugehörigen Zugmittels um das Umlenkrollenpaar unabhängig von der Schwenkstellung des Zwischenstücks ist. Dann hat ein Verschwenken des Zwischenstücks, d. h. ein Verschwenken des Instrumentenkopfes in Bezug auf den Instrumentenschaft keinerlei Einfluss auf die Werkzeugbewegung und die Werkzeugkräfte. Dies ist insbesondere bei manueller Betätigung des Instrumentes vorteilhaft aber auch bei robotischer Anbindung, da keine Bewegungskompensation erforderlich ist, was Rechen- und Motorkapazität erfordert.

Die Erfindung ist nachfolgend anhand eines in der Zeichnung dargestellten Ausführungsbeispiels näher erläutert. Es zeigen
- Fig. 1: in vereinfachter perspektivischer Darstellung das distale Schaftende mit Instrumentenkopf und den durch den Schaft führenden Zugmitteln,

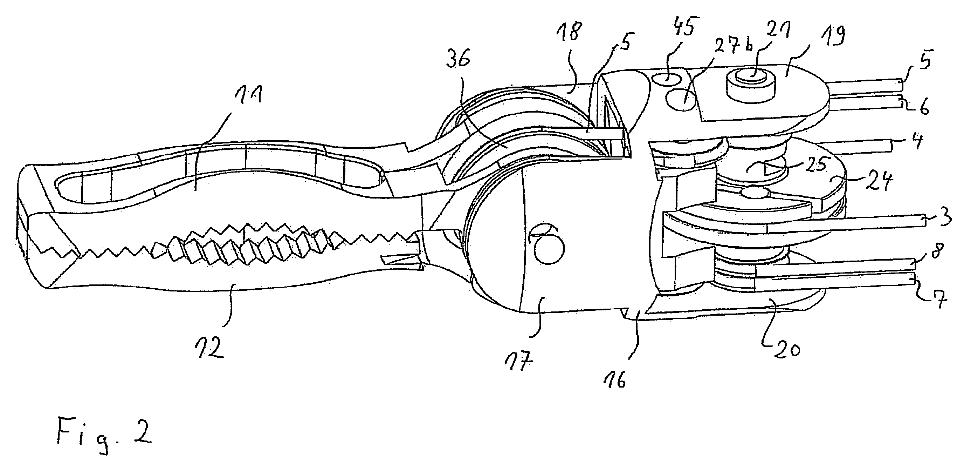

- Fig. 2: eine perspektivische Ansicht des distalen Instrumentenkopfes ohne Schaft,

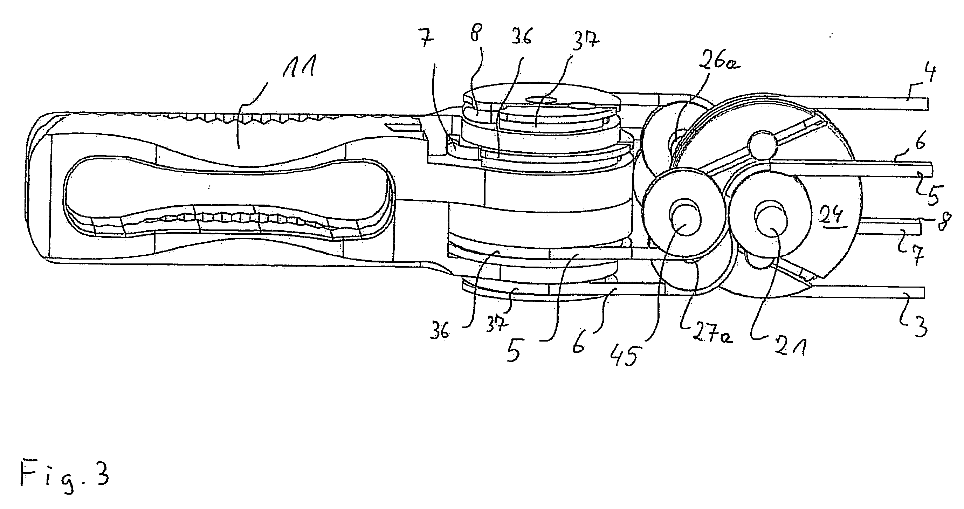

- Fig. 3: eine Ansicht des Instrumentenkopfes ohne Zwischenstück und Schaft,

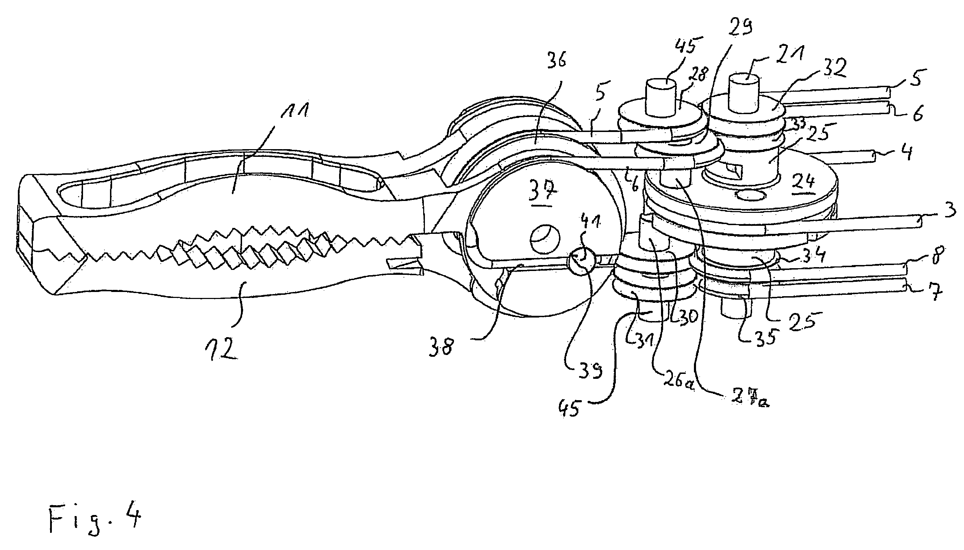

- Fig. 4: eine gegenüber Fig. 3 um 90° um die Längsachse gedrehte Darstellung,
- Fig. 5: in schematischer Darstellung das in Fig. 1 obere Maulteil mit den daran angebundenen Zugmitteln,
- Fig. 6: das in Fig. 1 dargestellte untere Maulteil mit den daran angebundenen Zugmitteln,
- Fig. 7: in perspektivischer Explosionsdarstellung die beiden Maulteile,
- Fig. 8: eine Ansicht auf das Schaftende mit Zwischenstück ohne Umlenkrollen und Maulteile,
- Fig. 9: eine gegenüber Fig. 8 um 90° gedrehte Ansicht,
- Fig. 10: eine Schnitt längs der Schnittlinie X-X in Fig. 9,
- Fig. 11: in vergrößerter perspektivischer Darstellung das Zwischenstück,
- Fig. 12: in perspektivischer Darstellung die Kurvenscheibe mit Welle und Zugmitteln zur Schwenkbewegung des Instrumentenkopfes,
- Fig. 13: eine Seitenansicht quer zur Schwenkachse der Bauteile gemäß Fig. 12 und
- Fig. 14: eine Seitenansicht in Richtung der Schwenkachse.

Von dem erfindungsgemäßen endoskopischen Instrument ist im Folgenden nur der distale Instrumententeil beschrieben und dargestellt. Der langgestreckte Schaft sowie die proximale Ausgestaltung, sei es als Handhabe zur unmittelbaren manuellen Betätigung durch den Operateur oder als robotischer Anschluss wie es bei dem aus US 6,312,435 B1 bekannten Instrument bekannt ist, zählt zum allgemeinen Stand der Technik und ist nicht Gegenstand der vorliegenden Erfindung.

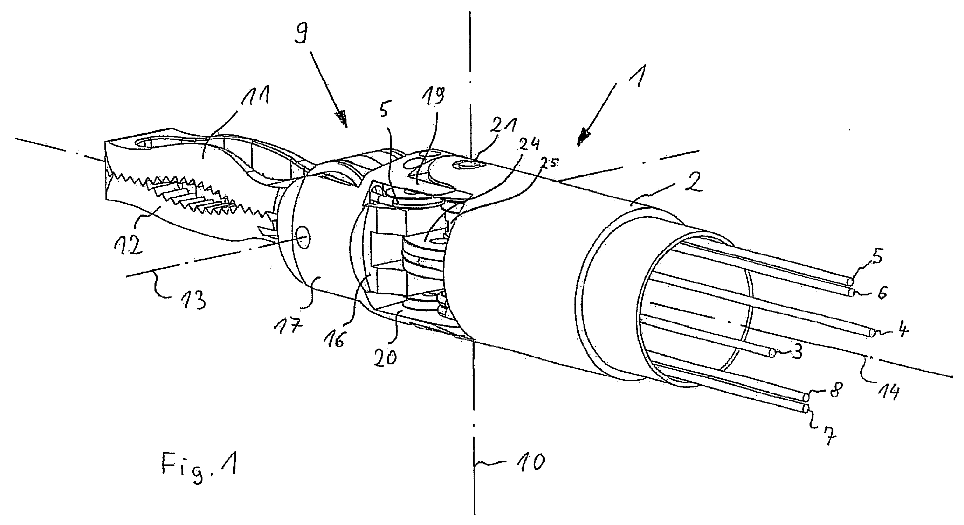

In Fig. 1 dargestellt ist das distale Schaftende 1 sowie die durch den Schaft 2 geführten Zugmittel in Form von Zugdrähten 3 - 8, welche durch den Schaft 2 hindurch bis zum proximalen Ende geführt sind und dort über Hebel, Rollen oder dergleichen zum Zwecke des Aufbringens von Zugkräften befestigt sind. Dabei dienen die Zugdrähte 3 und 4 zum Schwenken des Instrumentenkopfes 9 gegenüber dem distalen Schaftende 1 um eine Schwenkachse 10. Die Zugdrähte 5 und 6 dienen zum Verschwenken des in Fig. 1 dargestellten oberen Maulteils 11 eines Zangenmauls, dessen in Fig. 1 unteres Maulteil 12 durch die Zugdrähte 7 und 8 in seiner Schwenkbewegung steuerbar ist. Die Maulteile 11 und 12 sind um eine gemeinsame Achse 13 schwenkbar, welche bezogen auf die Instrumentenlängsachse 14 mit Abstand und um 90° gedreht zur Schwenkachse 10 angeordnet ist.

Alle im Folgenden gemachten Angaben bezogen auf die Längsachse 14 des Instrumentes beziehen sich auf die in Fig. 1 dargestellte, nicht abgewinkelte Anordnung von Instrumentenkopf 9 sowie den Maulteilen 11 und 12.

Der Instrumentenkopf 9 weist ein Zwischenstück 15 auf, das aus einem in der Außenkontur kreisrunden Grundkörper 16 aufgebaut ist und von dem sich distalwärts zwei um 180° bezogen auf die Längsachse 14 versetzte Schenkel 17 und 18 erstrecken, zwischen denen die Maulteile 11 und 12 schwenkbar gelagert sind, sowie proximalwärts zwei ebenfalls diametral angeordnete und bezogen auf die Achse 14 um 180° zueinander versetzt angeordnete Schenkel 19 und 20, die bezogen auf die Achse 14 um 90° versetzt zu den Schenkeln 17 und 18 angeordnet sind. Sowohl die Schenkel 17 und 18 als auch die Schenkel 19 und 20 sind nahe dem Außenumfang des Grundkörpers 16 angeordnet und setzen die im Querschnitt kreisrunde Schaftform des Schaftes 2 nach außen hin in diesen Bereichen fort.

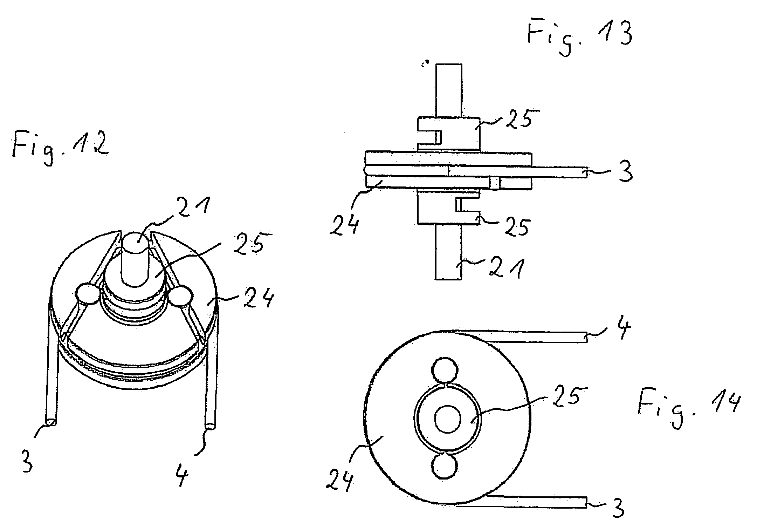

Die Schenkel 19 und 20 sind drehfest mit einer Welle 21 verbunden, welche drehbar in Ausnehmungen von Schenkeln gelagert, die am distalen Ende des Schaftes 2 vorgesehen sind. Auf der Welle 21 ist drehfest eine Kurvenscheibe 24 befestigt, über welche die Zugdrähte 3 und 4 verlaufen, die jeweils seitlich in der Scheibe mit ihren Enden festgelegt sind. Die Welle 21 trägt seitlich der Kurvenscheibe 24 noch zwei Distanzscheiben 25, die in der beschriebenen Ausführung einstückig mit der Kurvenscheibe 24 und der Welle 21 ausgebildet sind, die jedoch auch nach Art einer Unterlegscheibe drehbar auf der Welle 21 sitzen können. Es wird in diesem Zusammenhang insbesondere auf die Darstellungen in den Figuren 12 - 14 verwiesen, wo dies im Einzelnen dargestellt ist.

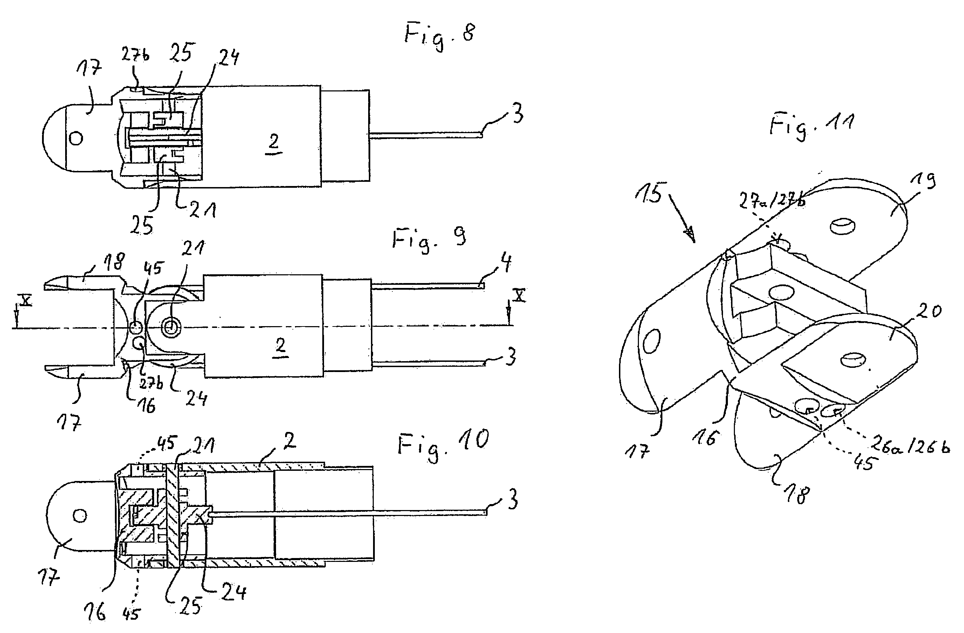

Die proximalwärts weisenden Schenkel 19 und 20 des Zwischenstücks 15 tragen im Bereich zwischen der Welle 21 und dem Grundkörper 16 noch drei parallele Achsen, nämlich eine, mit Abstand versetzt und parallel zur Welle 21, kurze Achse 45 sowie zwei jeweils seitlich versetzt dazu angeordnete ebenfalls kurze Achsen 26a und 27a. Die Achsen 45, 26a und 27a sind parallel zur Welle 21 angeordnet, die zentralen Achsen 45 liegen in Richtung der Längsachse 14 des Instrumentes gesehen hinter der Welle 21. Die Bohrungen für die kurzen Achsen 26a und 27a sind durch Blindstifte 26b bzw. 27b nach außen hin abgeschlossen. In welche Bohrungen die kurzen Achsen 26a und 27a sowie 45 in dem Zwischenstück 15 eingegliedert werden, ist anhand von Fig. 11 durch unterbrochene Linien angedeutet. Dabei ist in die entsprechende Bohrung beispielsweise zunächst die kurze Achse 26a einzusetzen und diese dann durch den Blindstift 26b zu verschließen, wie auf der anderen Seite zunächst die kurze Achse 27a einzuführen und dann durch den Blindstift 27b zu verschließen ist.

Die zentralen Achsen 45 tragen zwei Umlenkrollen 28 und 29, die kurzen Achsen 26a und 27a tragen jeweils eine Umlenkrolle 30 bzw. 31. Die Umlenkrollen 28 - 31 sind baugleich und frei drehbar gelagert, sie dienen zum Umlenken der Zugdrähte 5 - 8 und wirken zusammen mit weiteren vier baugleichen Umlenkrollen 32 - 35, die drehbar auf der Welle 21 seitlich der Distanzscheiben 25 jeweils paarweise gelagert sind. Dabei ist für jeden der Zugdrähte 5 - 8 ein Rollenpaar jeweils bestehend aus einer der Umlenkrollen 28 - 31 sowie einer auf der Welle 21 angeordneten Rollen 32 - 35 gebildet. Jedes Rollenpaar ist so angeordnet, dass unabhängig der Schwenkstellung des Instrumentenkopfes 9 zum Schaft 2 der Umschlingungswinkel eines Zugdrahtes um ein Rollenpaar stets konstant ist, d. h. in dem Maße, wie sich der Umschlingungswinkel einer auf der Welle 21 sitzenden Umlenkrolle 32 - 35 verringert, so vergrößert sich der Umschlingungswinkel der zugehörigen Umlenkrolle 28 - 31 in entsprechendem Maße und umgekehrt. Diese Anordnung bewirkt, dass die Stellung der Zugdrähte 5 - 8 bezogen auf die Schwenkstellung der damit verbundenen Maulteile 11 und 12 um die Achse 13 unabhängig von der Schwenkstellung des Instrumentenkopfes 9 um die Schwenkachse 10 ist.

Die Zangenmaulteile 11 und 12 sind frei beweglich um die Drehachse 13 gelagert und jeweils mit zwei Zugdrähten 5 und 6 bzw. 7 und 8 verbunden, wobei ein Zugdraht zum Schwenken eines Maulteils in Öffnungsrichtung und der andere Zugdraht zum Schwenken des Maulteils in Schließrichtung vorgesehen ist. Da jedem Maulteil 11 und 12 jeweils zwei Zugdrähte 5 und 6 bzw. 7 und 8 zugeordnet sind, können die Maulteile 11 und 12 nicht nur aufeinander zu und voneinander weg sondern auch gemeinsam geschwenkt werden, sodass das Werkzeug auch in einer von der Längsrichtung 14 abweichenden Richtung greifen kann.

Hierzu ist jedes Maulteil mit zwei Kurvenscheiben verbunden, nämlich mit einer nahe der Längsmittelachse 14 angeordneten großen Kurvenscheibe 36 und einer daneben angeordneten kleineren Kurvenscheibe 37. Die große Kurvenscheibe 36 weist einen deutlich größeren Radius als die kleine Kurvenscheibe 37 auf. Beide Kurvenscheiben sind so ausgebildet und angeordnet, dass sie in der in Fig. 1 dargestellten geraden Stellung bei geschlossenem Zangenmaul von einem Zugdraht 5 - 8 um mehr als 90° umschlungen sind. Sie weisen eine etwa in Verlängerung des Zangenmauls verlaufende seitliche Ausnehmung 38 auf, die kanalartig ausgebildet ist und zur Aufnahme des Zugseilendes vorgesehen ist. Quer hierzu ist eine Bohrung 39 vorgesehen, in der ein Querstift 41 angeordnet ist, in dem das Zugseilende festgelegt ist.

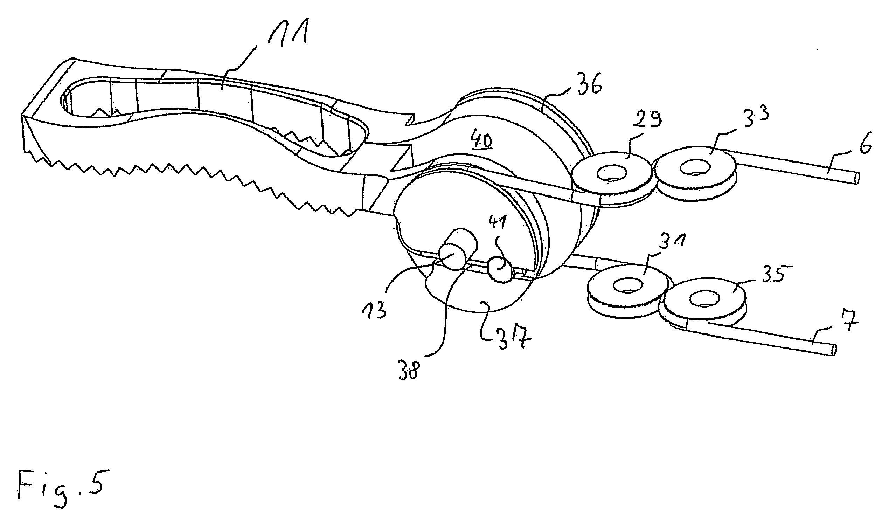

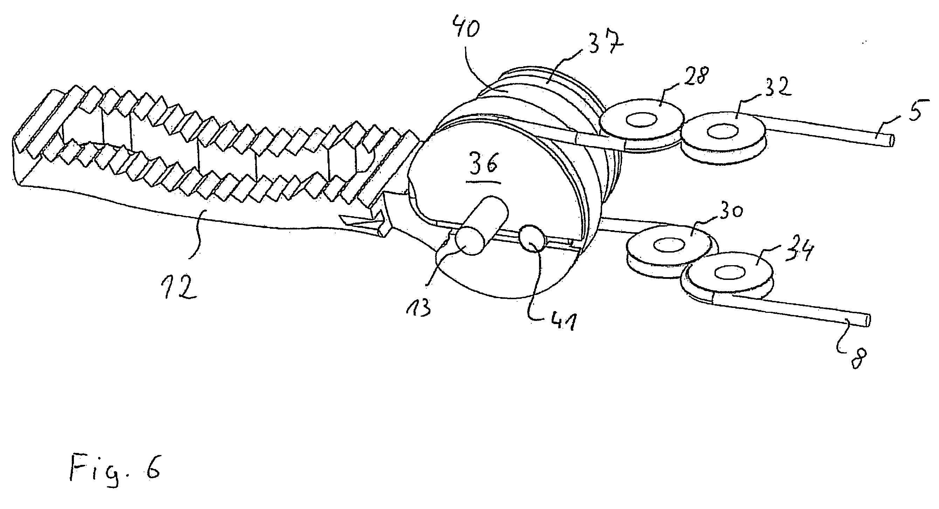

Bei der dargestellten Ausführungsform sind Zugdrähte, welche das jeweilige Zangenmaulteil 11 bzw. 12 auf das andere Zangenmaulteil 12 bzw. 11 zubewegen, d. h. das Maulteil in Schließrichtung beaufschlagen, an den großen Kurvenscheiben 36 festgelegt, die nahe der Längsmittelachse 14 etwa mittig zwischen den Schenkeln 17 und 18 angeordnet sind. Die im Durchmesser kleineren Kurvenscheiben 37 sind mit den Zugdrähten der Maulteile 11 und 12 verbunden, die zum Öffnen des Zangenmauls dienen. Durch diese Anordnung ist es möglich, bei gleicher Zugkraft auf die Zugdrähte 5 und 6 bzw. 7 und 8 eine höhere Kraft der Zangenmaulteile 11 und 12 in Schließrichtung des Mauls als in Öffnungsrichtung zu erzeugen, da aufgrund des größeren Radius der großen Kurvenscheiben 36 dort ein größeres Moment erzeugt wird, als bei gleichgroßer Krafteinleitung auf die kleineren Umlenkscheiben 37 erzeugt wird. Wie insbesondere die Figuren 5 und 6 verdeutlichen, sind die großen Kurvenscheiben 36 innenliegend, also dort, wo bezogen auf den Scheibendurchmesser der größte Freiraum innerhalb der Schaftkontur gegeben ist, wohingegen die kleineren Kurvenscheiben 37 weiter außen liegen, dort, wo der Freiraum aufgrund der Krümmung geringer ist.

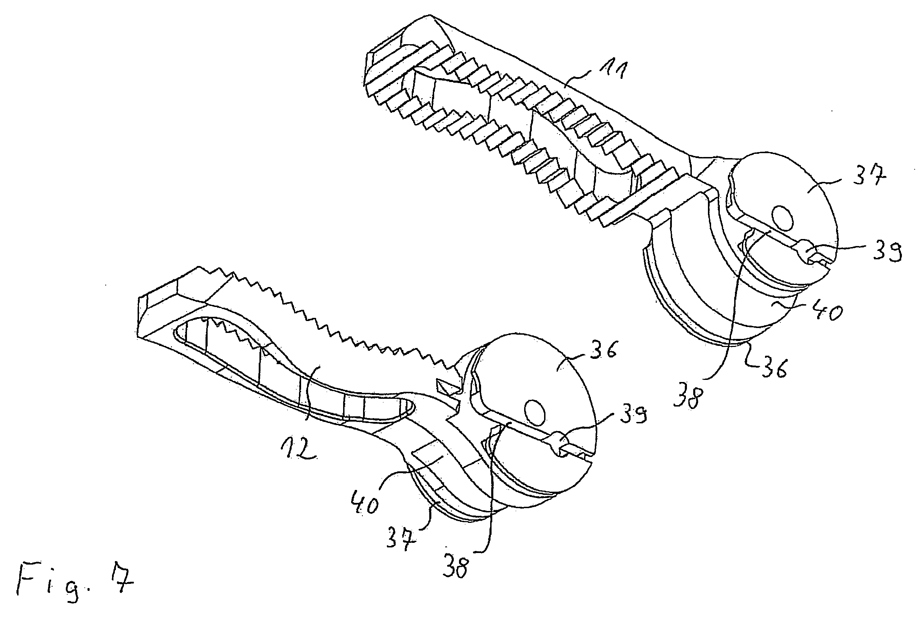

Wie insbesondere die Fig. 7 verdeutlicht, sind die Maulteile 11 und 12 mit den zugehörigen Kurvenscheiben 36 und 37 im dargestellten Ausführungsbeispiel identisch ausgebildet, d. h. es können zwei identische Bauteile Verwendung finden. Dabei ist im Bereich der Kurvenscheiben zwischen der großen Kurvenscheibe 36 und der kleineren Kurvenscheibe 37 ein Freiraum 40 gebildet, der so bemessen ist, dass bei Drehung des Bauteils um 180° um die Längsachse die große Kurvenscheibe 36 eines Maulteils 12 zwischen die beiden Kurvenscheiben 36, 37 des anderen Maulteils 11 eingegliedert werden kann. Dann liegen die beiden großen Kurvenscheiben 36 jeweils unmittelbar neben der Längsmittelachse 14 und die kleineren Kurvenscheiben 37 mit Abstand daneben.

Der vorbeschriebene Instrumentenkopf kann durch Aufbringen einer Zugkraft an den Zugdrähten 3 oder 4 um die Achse 10 in zwei Richtungen geschwenkt werden. Durch Aufbringen von Zugkräften an den Zugdrähten 6 und 8 kann das Zangenmaul geöffnet werden, durch Aufbringen von Zugkräften an den Zugdrähten 5 und 7 wird dieses geschlossen. Werden Zugkräfte an den Zugdrähten 6 und 7 bzw. 5 und 8 gleichzeitig aufgebracht, so schwenken beide Maulteile in dieselbe Richtung um die Achse 13 ohne ihre Winkelstellung zueinander zu verändern.

### Bezugszeichenliste

- 1: distales Schaftende
- 2: Schaft
- 3: Zugdraht
- 4: Zugdraht
- 5: Zugdraht
- 6: Zugdraht
- 7: Zugdraht
- 8: Zugdraht
- 9: Instrumentenkopf
- 10: Schwenkachse
- 11: oberes Maulteil in Fig. 1
- 12: unteres Maulteil in Fig. 1
- 13: Schwenkachse der Maulteile
- 14: Längsachse des Instruments in Ausgangslage gemäß Fig. 1, Schaftachse
- 15: Zwischenstück
- 16: Grundkörper von 15
- 17: distalwärts gerichteter Schenkel
- 18: distalwärts gerichteter Schenkel
- 19: proximalwärts gerichteter Schenkel
- 20: proximalwärts gerichteter Schenkel
- 21: Welle
- 22: Schenkel am distalen Schaftende
- 23: Schenkel am distalen Schaftende
- 24: Kurvenscheibe
- 25: Distanzscheiben
- 26a: kurze Achse
- 26b: Blindstift
- 27a: kurze Achse
- 27b: Blindstift
- 28: Umlenkrollen
- 29: Umlenkrollen
- 30: Umlenkrollen
- 31: Umlenkrollen
- 32: Umlenkrollen auf Welle 21
- 33: Umlenkrollen auf Welle 21
- 34: Umlenkrollen auf Welle 21
- 35: Umlenkrollen auf Welle 21
- 36: große Kurvenscheibe
- 37: kleine Kurvenscheibe
- 38: kanalartige Ausnehmung
- 39: Querbohrung
- 40: Freiraum zwischen den Kurvenscheiben
- 41: durchbohrter Querstift
- 45: zentrale kurze Achsen

## Patentansprüche

1. Endoskopisches Instrument mit einem Schaft (2) und mit einem am distalen Schaftende (1) angeordneten Instrumentenkopf (9), welcher ein Werkzeug mit zwei zueinander schwenkbaren Maulteilen (11, 12) aufweist, die über im Schaft (2) geführte Zugmittel (5 - 8) vom proximalen Instrumentenende aus steuerbar sind, wobei mindestens ein Maulteil (11, 12) zwei Kurvenscheiben (36, 37) aufweist, an denen je ein Zugmittel (5 - 8) angreift, **dadurch gekennzeichnet, dass** mindestens einem Maulteil (11, 12) zwei Kurvenscheiben (36, 37) unterschiedlicher radialer Größe zugeordnet sind, wobei die radial größere Kurvenscheibe (36) näher zur Längsmittelachse (14) des Werkzeugs angeordnet ist als die radial kleinere Kurvenscheibe (37).

2. Instrument nach Anspruch 1, **dadurch gekennzeichnet, dass** die radial größere Kurvenscheibe (36) dem Zugmittel (6, 8) zum Schließen und die radial kleinere Kurvenscheibe (37) dem Zugmittel (5, 7) zum Öffnen des Maulteil (11, 12) zugeordnet ist.

3. Instrument nach Anspruch 1 oder 2, **dadurch gekennzeichnet, dass** die Kurvenscheiben (36, 37) vorzugsweise kreisförmig ausgebildet und auf der gemeinsamen Schwenkachse (13) der Maulteile (11, 12) angeordnet sind.

4. Instrument nach einem der vorhergehenden Ansprüche, **dadurch gekennzeichnet, dass** die Zugmittel (5 - 8) jeweils an den Kurvenscheiben (36, 37) festgelegt sind und diese um mehr als 90° umschlingen.

5. Instrument nach einem der vorhergehenden Ansprüche, **dadurch gekennzeichnet, dass** ein Maulteil (11, 12) einstückig mit den zwei zugehörigen Kurvenscheiben (36, 37) ausgebildet ist.

6. Instrument nach einem der vorhergehenden Ansprüche, **dadurch gekennzeichnet, dass** die beiden Maulteile (11, 12) identisch ausgebildet sind.

7. Instrument nach einem der vorhergehenden Ansprüche, **dadurch gekennzeichnet, dass** die Maulteile (11, 12) in einem Zwischenstück (15) gelagert sind, welches Teil des Instrumentenkopfes (9) bildet und das um eine Schwenkachse (10) schwenkbar am distalen Schaftende (1) angeordnet ist.

8. Instrument nach Anspruch 7, **dadurch gekennzeichnet, dass** die Schwenkachse (10) des Zwischenstücks (15) mit Abstand und quer zur Schwenkachse (13) der Maulteile (11, 12) angeordnet ist.

9. Instrument nach Anspruch 7 oder 8, **dadurch gekennzeichnet, dass** das Zwischenstück (15) einen Grundkörper (16) aufweist, von dem sich zwei Schenkel (17, 18) distalwärts erstrecken, welche die Maulteile (11, 12) mit ihrer Schwenkachse (13) aufnehmen, und von dem sich in Achsrichtung (14) um 90° versetzt dazu zwei Schenkel (19, 20) proximalwärts erstrecken, welche die Schwenkachse (10) des Zwischenstücks (15) und darauf angeordnete Bauteile (24, 25) aufnehmen.

10. Instrument nach Anspruch 9, **dadurch gekennzeichnet, dass** die proximalwärts gerichteten Schenkel (19, 20) drehfest mit einer Welle (21) verbunden sind, auf der drehfest eine Kurvenscheibe (24) angeordnet ist, an welcher Zugmittel (3, 4) zur Steuerung der Schwenkstellung des Zwischenstücks (15) angreifen.

11. Instrument nach Anspruch 10, **dadurch gekennzeichnet, dass** zwischen den proximalwärts gerichteten Schenkeln (19, 20) für jedes zu einem Maulteil (11, 12) führende Zugmittel (5 - 8) ein Umlenkrollenpaar (28 - 35) drehbar gelagert ist, vom dem jeweils eine Umlenkrolle (32 - 35) auf der Welle (21) und die andere (28 - 31) versetzt dazu zwischen Welle (21) und Grundkörper (16) angeordnet ist.

12. Instrument nach Anspruch 11, **dadurch gekennzeichnet, dass** jedes Umlenkrollenpaar (28 - 35) so zueinander angeordnet ist, dass der Umschlingungswinkel des zugehörigen Zugmittels (5 - 8) um das Umlenkrollenpaar (28 - 35) unabhängig von der Schwenkstellung des Zwischenstücks (15) ist.

## Claims

1. An endoscopic instrument with a shank (2) and with an instrument head (9) which is arranged at the distal shank end (1) and which comprises a tool with two jaw parts (11, 12) which are pivotable to one another and which can be controlled from the proximal instrument end via pull means (5-8) led in the shank (2), wherein at least one jaw part (11, 12) comprises two curved disks (36, 37), on which a pull means (5-8) engages in each case, **characterised in that** two curved disks (36, 37) of a different radial size are assigned to at least one jaw part (11, 12), wherein the radially larger curved disk (36) is arranged closer to the longitudinal middle axis (14) of the tool than the radially smaller curved disk (37).

2. An instrument according to claim 1, **characterised in that** the radially larger curved disk (36) is assigned to the pull means (6, 8) for the closure, and the radially smaller curved disk (37) to the pull means (5, 7) for opening the jaw part (11, 12).

3. An instrument according to claim 1 or 2, **characterised in that** the curved disks (36, 37) are preferably designed in a circular manner and are arranged on the common pivot (13) of the jaw parts (11, 12).

4. An instrument according to one of the preceding claims, **characterised in that** the pull means (5 - 8) are each fastened on the curved disks (36, 37) and wrap around these by more than 90°.

5. An instrument according to one of the preceding claims, **characterised in that** a jaw part (11, 12) is designed as one piece with the two associated curved disks (36, 37).

6. An instrument according to one of the preceding claims, **characterised in that** the two jaw parts (11, 12) are designed in an identical manner.

7. An instrument according to one of the preceding claims, **characterised in that** the jaw parts (11, 12) are mounted in an intermediate piece (15) which forms part of the instrument head (9) and which is pivotably arranged about a pivot axis (10), at the distal shank end (1).

8. An instrument according to claim 7, **characterised in that** the pivot axis (10) of the intermediate piece (15) is arranged at a distance and transversely to the pivot axis (13) of the jaw parts (11, 12).

9. An instrument according to claim 7 or 8, **characterised in that** the intermediate piece (15) comprises a base body (16), from which two limbs (17, 18) receiving the jaw parts (11, 12) with their pivot axis (13) extend distally, and from which two limbs (19, 20) receiving the pivot axis (10) of the intermediate piece (15) and components (24, 25) arranged thereon, extend proximally in the axis direction (14) offset by 90° thereto.

10. An instrument according to claim 9, **characterised in that** the proximally directed limbs (19, 20) are connected to a shaft (21) in a rotationally fixed manner, wherein a curved disk (24), on which pull means (3, 4) engage for the control of the pivot position of the intermediate piece (15), is arranged on said shaft (21) in a rotationally fixed manner.

11. An instrument according to claim 10, **characterised in that** a deflection roller pair (28 - 35) is rotatably mounted between the proximally directed limbs (19, 20), for each pull means (5 -8) leading to a jaw part (11, 12), of which deflection roller pairs in each case one deflection roller (32 - 35) is arranged on the shaft (21) and the other (28-31) is arranged offset thereto between the shaft (21) and the base body (16).

12. An instrument according to claim 11, **characterised in that** each deflection roller pair (28 -35) is arranged to one another such that the wrap angle of the associated pull means (5 - 8) about the deflection roller pair (28 - 35) is independent of the pivot position of the intermediate piece (15).

## Revendications

1. Instrument endoscopique avec une tige (2) et une tête d'instrument (9) disposée à une extrémité de tige (1) distale, laquelle tête comprend un outil avec deux mâchoires (11, 12) aptes à pivoter l'une vers l'autre qui sont conformées pour être commandées à partir de l'extrémité d'instrument proximale à l'aide de moyens de traction (5 - 8) guidés à l'intérieur de la tige (2), au moins une mâchoire (11, 12) comprenant deux disques à came (36, 37) à chacun desquels est attaché un moyen de traction (5 - 8), **caractérisé en ce qu'**à au moins une mâchoire (11, 12) sont associés deux disques à came (36, 37) de tailles radiales différentes, le disque à came (36) radialement plus grand étant disposé plus proche de l'axe central longitudinal (14) de l'outil que le disque à came (37) radialement plus petit.

2. Instrument selon la revendication 1, **caractérisé en ce que** le disque à came (36) radialement plus grand est associé au moyen de traction (6, 8) pour fermer, et le disque à came (37) radialement plus petit est associé au moyen de traction (5, 7) pour ouvrir la mâchoire (11, 12).

3. Instrument selon la revendication 1 ou 2, **caractérisé en ce que** les disques à came (36, 37) ont de préférence une forme circulaire et sont disposés sur l'axe de pivotement commun (13) des mâchoires (11, 12).

4. Instrument selon l'une des revendications précédentes, **caractérisé en ce que** les moyens de traction (5 - 8) sont attachés chacun aux disques à cames (36, 37) et les enveloppent sur plus que 90°.

5. Instrument selon l'une des revendications précédentes, **caractérisé en ce qu'**une mâchoire (11, 12) est réalisée en une seule pièce avec les deux disques à came correspondantes (36, 37).

6. Instrument selon l'une des revendications précédentes, **caractérisé en ce que** les deux mâchoires (11, 12) sont formées de façon identique.

7. Instrument selon l'une des revendications précédentes, **caractérisé en ce que** les mâchoires (11, 12) sont montées sur une pièce intermédiaire (15) qui fait partie de la tête d'instrument (9) et qui est disposée, de manière pivotante autour d'un axe de pivotement (10), à l'extrémité de tige (1) distale.

8. Instrument selon la revendication 7, **caractérisé en ce que** l'axe de pivotement (10) de la pièce intermédiaire (15) est disposé à distance de, et transversalement par rapport à, l'axe de pivotement (13) des mâchoires (11, 12).

9. Instrument selon la revendication 7 ou 8, **caractérisé en ce que** la pièce intermédiaire (15) comprend un corps de base (16) à partir duquel s'étendent deux branches (17, 18) en direction distale qui reçoivent les mâchoires (11, 12) avec leur axe de pivotement (13), et à partir duquel s'étendent deux branches (19, 20), décalées par rapport à celles-ci de 90° autour de l'axe (14), en direction proximale qui reçoivent l'axe de pivotement (10) de la pièce intermédiaire (15) ainsi que des composants (24, 25) disposés sur celle-ci.

10. Instrument selon la revendication 9, **caractérisé en ce que** les branches (19, 20) orientées en direction proximale sont solidaires en rotation avec un axe (21) sur lequel est disposé, de manière solidaire en rotation, un disque à came (24) sur lequel sont attachés des moyens de traction (3, 4) pour la commande de la position de pivotement de la pièce intermédiaire (15).

11. Instrument selon la revendication 10, **caractérisé en ce que**, entre les branches (19, 20) orientées en direction proximale, est disposée de manière rotative, pour chaque moyen de traction allant vers une mâchoire (11, 12), une paire de rouleaux de renvoi (28 - 35) dont un rouleau de renvoi (32 - 35) respectif est disposé sur l'axe (21) et dont l'autre (28 - 31) est disposé, de manière décalée par rapport à celui-ci, entre l'axe (21) et le corps de base (16).

12. Instrument selon la revendication 11, **caractérisé en ce que** les rouleaux de chaque paire de rouleaux de renvoi (28 - 35) sont disposés l'un par rapport à l'autre de façon que l'angle d'enveloppement du moyen de traction correspondant (5 - 8) autour de la paire de rouleaux de renvoi (28 - 35) est indépendant de la position de pivotement de la pièce intermédiaire (15).
